Europäisches Patentamt

European Patent Office

Office européen des brevets

(11) Publication number: **0 409 086 A1**

# EUROPEAN PATENT APPLICATION

(21) Application number: **90113362.9**

(51) Int. Cl.5: **C07C 43/04, C07C 41/01**

(22) Date of filing: **12.07.90**

(30) Priority: **18.07.89 US 381450**

(43) Date of publication of application:
**23.01.91 Bulletin 91/04**

(84) Designated Contracting States:
**BE DE ES FR GB IT NL**

(71) Applicant: **AIR PRODUCTS AND CHEMICALS, INC.**
**7201 Hamilton Boulevard**
**Allentown, PA 18195-1501(US)**

(72) Inventor: **Hsiung, Thomas Hsiao-Ling**
**4727 Glenwood Circle**
**Emmaus, PA 18049(US)**
Inventor: **White, James Ferguson**
**4970 Briarwood Drive**
**Macungie, PA 18062(US)**
Inventor: **Lewnard, John Joseph**
**R.D. No.41**
**Emmaus, PA 18049(US)**

(74) Representative: **Dipl.-Ing. Schwabe, Dr. Dr. Sandmair, Dr. Marx**
**Stuntzstrasse 16**
**D-8000 München 80(DE)**

(54) **One-step liquid phase process for dimethyl ether synthesis.**

(57) A one-step process is disclosed for the production of dimethyl ether, with varying amounts of co-product methanol, from synthesis gas. In the process, synthesis gas with a wide range of compositions of $H_2$, CO, $CO_2$, and other species is contacted with a catalyst or mixture of catalysts suspended in an inert liquid in a three phase reactor system. In situations where the feed gas is relatively rich in CO and poor in $H_2$, $H_2O$ may be co-fed to enhance productivity. The catalyst, which comprises both a methanol synthesis component and a dehydration (ether forming) component, can be in the form of pellets or powder, depending on the mode of operation for the reactor.

EP 0 409 086 A1

# ONE-STEP LIQUID PHASE PROCESS FOR DIMETHYL ETHER SYNTHESIS

This application is a continuation-in-part of U.S.S.N 07/143,799 filed on January 14, 1988.

## TECHNICAL FIELD

The present invention relates to a process for the production of dimethyl ether. More specifically, the present invention relates to a process for the direct production of dimethyl ether from synthesis gas using a three-phase reactor system.

## BACKGROUND OF THE INVENTION

Conversion of synthesis gas to dimethyl ether requires three steps. Conventionally, synthesis gas is produced by reforming hydrocarbon or gasifying a carbon source such as coal or coke. Since this later synthesis gas usually is too rich in CO to be used directly for dimethyl ether synthesis, an intermediate step is needed for conventional dimethyl ether manufacture. Consequently, the first step in the dimethyl ether synthesis is to adjust the composition of the synthesis gas via the water-gas shift reaction:

$$CO + H_2O \rightleftharpoons CO_2 + H_2 \qquad (1)$$

After the ratio of hydrogen to carbon oxides has been adjusted, the gas is reacted to produce methanol (MeOH):

$$CO + 2 H_2 \rightleftharpoons CH_3OH \qquad (2)$$

Finally, methanol is dehydrated to form dimethyl ether (DME):

$$2 (CH_3OH) \rightarrow CH_3OCH_3 + H_2O \qquad (3)$$

The shift and methanol synthesis reactions are equilibrium limited and exothermic. Moreover, the catalysts for both reactions are subject to severe deactivation when overheated. To avoid thermodynamic limitations and excessive catalyst deactivation, conventional gas phase reactors must be run at low per-pass conversions to maintain reactor temperature. Consequently, overall conversion of carbon monoxide to dimethyl ether is limited.

Multi-step processes, which use separate reactors for each reaction, can not exploit the potential synergism of the three reactions. If these three reactions could be conducted simultaneously, methanol synthesis would drive the forward shift reaction, and dimethyl ether synthesis would drive both the methanol and shift reaction. Consequently, a one-step process is more flexible and can operate under a wider range of conditions than a multi-step process. In addition, multi-step processes require separate reactors, heat exchangers, and associated equipment for each reaction.

A single-step gas phase process would generally require less equipment than multi-step gas processes. However, a single-step gas-phase process would still suffer from a large reactor exotherm due to the high net heat of reaction. Hence, low per-pass conversions would be required to maintain reactor temperature to avoid a short catalyst life due to the large temperature rises associated with these reactions. Since the reactor is not isothermal, there are often severe equilibrium limitations on per pass reactant conversions.

Much of the prior art for dimethyl ether synthesis focuses on processes using improved catalysts to run shifted syngas ($H_2$/CO greater than or equal to 1). Examples include U.S. Patents 4,417,000; 4,423,155; 4,520,216; 4,590,176; and 4,521,540. These processes all run in the gas phase, and may be considered multi-step processes in that they all require the feed be shifted via Reaction (1).

Single-step gas-phase processes have been disclosed by Mobil Corp. and Haldor-Topsoe. For example, U.S. Patent 4,011,275 assigned to Mobil Corp. discloses a gas-phase process for co-production of methanol and dimethyl ether with $H_2$ deficient syngas feeds. Although there are no examples in the patent, the process is claimed to be useful for improving conversion of synthesis gas. U.S. Patent 4,341,069 discloses a gas-phase process for dimethyl ether production to be used in conjunction with an integrated gasification combined cycle power plant. Examples in the patent show that the catalyst requires frequent regeneration, in some cases on a daily basis. Another gas-phase process is described in U.S. Patent 4,481,305, however, this process is restricted to operation within a narrow range of CO/CO_2 ratio in the feed gas. It should be noted that efficient heat removal to maintain reactor temperature is generally not discussed in these patents. Fujimoto et al discussed in Chem. Letters, p.2051 (1984) the chemistry of the gas-phase one-step processes.

Combined methanol/dimethyl ether synthesis in the liquid phase has been reported by several workers. Sherwin and Blum, in their paper: "Liquid Phase Methanol Interim Report, May 1978", prepared for the Electric Power Research Institute, attempted to modify the liquid phase methanol process for co-production of dimethyl ether by adding acid catalyst components to the system. They observed only traces of dimethyl ether, and concluded that the attempt was unsuccessful. Daroda, et al, J.C.S. Chem. Comm. p.1101 (1980), reported a broad slate of products for reactions of syngas with Fe in 2-methoxyethanol. However, in their system the solvent appears to act as a reactant, and the catalyst produces many side products. Consequently, neither earlier liquid phase process was economic.

## SUMMARY OF THE INVENTION

The present invention is a process for direct production of dimethyl ether, with varying amounts of co-product methanol, from wide varieties of synthesis gas. Basically, the present invention is an improvement to a process for the synthesis of dimethyl ether from a synthesis gas comprising hydrogen, carbon monoxide and carbon dioxide, wherein the synthesis gas is reacted in the presence of a solid methanol synthesis catalyst to produce methanol and then the produced methanol is reacted in the presence of a solid dehydration catalyst to produce dimethyl ether. The improvement comprises contacting and reacting the synthesis gas in the presence of a solid catalyst system which comprises both a methanol synthesis component and a dehydration (ether forming) component. The catalyst system can be a single catalyst or a mixture of catalysts in a liquid medium in a three phase (liquid phase) reactor system. In order to produce significant amounts of dimethyl ether, the process operation must be controlled so as to maintain a minimum effective methanol rate of at least 1.0 gmol methanol/(kg catalyst ● hr). The effective methanol rate is defined as the sum of the kinetic rate of methanol formation plus two times the kinetic rate of dimethyl ether formation.

The reactor system can either be a single three phase reactor or a series of staged three phase reactors. Even though the process of the present invention can be carried out in a series of the staged three phase reactors, the dimethyl ether synthesis is carried out in a single step, i.e., all three reactions in the synthesis route are being driven simultaneously.

The process of the present invention can be operated in either an ebullated bed mode with a granulated (shaped pellet) catalyst having a typical pellet diameter of about 1/8" to 1/4", or a slurry mode with a powdered catalyst having a particle size of less than 200 microns. The concentration of catalyst in the liquid medium is in the range from about 5 wt% to about 60 wt%. As stated earlier, the catalyst utilized in the process should comprise both a methanol synthesis component and a dehydration component. The methanol synthesis component can for example comprise a typical copper methanol synthesis catalyst. The dehydration component can be selected from the group consisting of alumina, silica-alumina, zeolites (e.g., ZSM-5), solid acids (e.g., boric acid), solid acid ion exchange resins (e.g., perfluorinated sulfonic acid) and mixtures thereof.

The preferred operating conditions of the process are a pressure range from about 200 psig to about 2000 psig, more preferably from about 400 psig to about 1000 psig; a temperature range from about 200°C to about 350°C; and a space velocity in excess of 50 standard liters of synthesis gas per kilogram of catalyst per hour, more preferably in the range from about 1000 to about 10,000 standard liters of synthesis gas per kilogram of catalyst per hour.

The process is particularly useful for higher CO content synthesis gases, even where the concentration of carbon monoxide in the synthesis gas is in excess of 50 vol%.

The process can also comprise a further step of adding water to the three phase reactor as a co-feed. The water can be added as a liquid or a vapor. The addition of water is particularly benecifical when the concentration of hydrogen in the synthesis gas is less than 10 vol%.

The process of the present invention is particularly suited for use in an integrated gasification combined cycle power plant for the production of electrical energy, wherein the process of the present invention would produce a storable fuel for peak-shaving.

## BRIEF DESCRIPTION OF THE DRAWING

3

Figure 1 is a plot of the rate of dimethyl ether formation versus the effective methanol rate.

Figure 2 is a plot of dimethyl ether selectivity versus the effective methanol rate.

## DETAILED DESCRIPTION OF THE INVENTION

The present invention is a single step process for the direct synthesis of dimethyl ether from synthesis gas with or without co-product methanol in the liquid phase. Selectivity for dimethyl ether and methanol can be optimized by varying reaction conditions and/or catalyst compositions to suit the process application.

The process uses a single catalyst or a physical mixture of catalysts which can be in the form of shaped pellets or in the form of a fine powder, depending on the mode of operation. Many types of catalysts are known in the literature for each individual reaction of the process, and these can be mixed in various proportions in the reactor. Alternately, a single catalyst component may be used to facilitate all three reactions. However, the catalyst utilized in the process should have a methanol synthesis component and a dehydration component. An example of a methanol synthesis catalytic component is copper. Examples of a dehydration or ether forming component are alumina, silica-alumina, zeolites, solid acids such as boric acid, and solid acid ion exchange resins such as perfluorinated sulfonic acids.

Catalyst concentrations in the liquid medium can vary from very dilute, i.e., 5 wt%, to very concentrated, i.e., 60 wt% or higher. The catalyst is contained in an inert oil, such as a paraffinic hydrocarbon or a hydrocarbon blend. Other types of liquids are known to work for liquid phase processes, for example, oxygenated species such as alcohols, ethers, and polyethers. These oxygenated liquids should be inert, and have a boiling point for single component liquids or boiling range for blended liquids between $150°C$ and $450°C$.

In the process of the present invention, synthesis gas is introduced into the reactor and contacts the catalyst contained in the liquid medium. The synthesis gas is typically comprised of $H_2$, $CO$, $CO_2$, and often inert species such as $N_2$ and $CH_4$. The composition of the gas can vary widely, as shown in the Examples. Depending on feed concentrations of $H_2$, $CO$, and $CO_2$, it may be advantageous to co-feed $H_2O$ either as a liquid or vapor to the process in order to adjust the gas composition via the shift reaction. This water addition is particularly beneficial when the concentration of hydrogen in the synthesis gas is less than 10 vol%. In addition, it may be advantageous to remove $CO_2$ from the feed gas in order to affect the dimethyl ether product selectivity. The removal of $CO_2$ can be accomplished by any conventional means, e.g., pressure swing adsorption or absorption using $CO_2$ selective solvents such as amines. The feed gas can be composed entirely of fresh feed in a once-through application, or it may be composed of a mixture of fresh feed and recycled gas.

Process conditions can vary widely, depending on operating conditions and type of reactor. Pressure ranges from ambient to high pressure, since increasing pressure typically enhances synthesis. Preferred pressures range from 200 to 2000 psig, and higher; more preferably 400 to 1000 psig. Temperature can range from $200°C$ to $350°C$, and preferably from $225°C$ to $300°C$. Space velocities, measured as standard liters of synthesis gas per kilogram of catalyst per hour can range from 50 to 50,000 and higher, depending on the cost of downstream separation of the dimethyl ether and methanol products; the most preferable range is between 1,000 and 20,000 standard liters of synthesis gas per kilogram of catalyst per hour.

Process conditions and yields are illustrated in the following series of examples, which describe the use of various catalyst mixtures as well as single components, and co-feed of $H_2O$. All runs were made in either a 300 cc or a 1 liter stainless steel autoclave with feed and product gas analysis via gas chromatograph.

Although the following examples were carried out in a single three phase reactor, the process of the present invention can be carried out in a series of staged three phase reactors. Process conditions for the different reactors can be varied, however, the reactor conditions are not varied from reactor to reactor to isolate and accomplish a single reaction in the synthesis route. The process of the present invention is accomplished by simultaneously carrying out the three reactions of the dimethyl ether synthesis route.

EXAMPLE I

The first series of experiments were performed with a so-called balanced gas (55% $H_2$, 19% $CO$, 5% $CO_2$, 21% $N_2$) at $250°C$ and 800 psig. A 25 wt% slurry consisting of 20 grams BASF S3-85 methanol synthesis catalyst and 20 grams of 200 mesh Catapal® high purity alumina was prepared in degassed Witco 70 oil. Results are shown in Table I; methanol and dimethyl ether were the only detectable products.

Comparison of CO conversions for methanol alone and dimethyl ether shows that the single-step dimethyl ether process is more efficient in overall CO conversion than production of methanol alone. In fact, Runs 1 and 2 show CO conversions greater than the thermodynamic maximum conversion for methanol alone.

$CO_2$ was found to have a major impact on dimethyl ether formation. Run 5, with no inlet $CO_2$, showed substantially higher dimethyl ether productivity and selectivity than Run 3. This comparison illustrates the potential for $CO_2$ removal from the feed gas.

Table 1

| Run | Feed | Temp (°C) | GHSV (s-l/kg catal•hr) | Productivity (gmol/kg catal•hr) | | DME/MeOH Selectivity (mol%/mol%) | CO Conversion (mol %)[1] |
|---|---|---|---|---|---|---|---|
| | | | | DME | MeOH | | |
| 1 | Bal | 250 | 1500 | 2.3 | 1.6 | 60/40 | 69 (44) |
| 2 | Bal | 250 | 2500 | 3.2 | 2.6 | 55/45 | 55 (40) |
| 3 | Bal | 250 | 2750 | 2.8 | 3.0 | 49/51 | 52 (39) |
| 4 | Bal | 250 | 5000 | 3.6 | 4.2 | 46/54 | 36 (32) |
| 5 | Bal[2] | 250 | 2750 | 4.7 | 1.5 | 76/24 | 65 |

Note 1: Numbers in parentheses indicate carbon monoxide conversion observed for methanol synthesis in the absence of dimethyl ether synthesis.
Note 2: Balanced gas with no inlet $CO_2$ (57% $H_2$, 20% CO, 0% $CO_2$, 23% $N_2$)

EXAMPLE II

To illustrate the use of a different catalyst mixture, and a different gas feed, a second series of experiments was made with 20 grams BASF S3-85 methanol catalyst and 40 grams Davison Silica/Alumina in degassed Witco 70 oil. Ten conditions were run with CO-rich and balanced gas at temperatures of 250°C and ~265°C, and pressure of 800 psig. Results are shown in Table 2. Balanced gas has the same composition as the earlier examples, and CO-rich gas is comprised of 35% $H_2$, 51% CO, 13% $CO_2$, and 1% $N_2$. Space velocities and productivities are based on the total oxide catalyst charged to the system.

Table 2

| Run | Feed | Temp (°C) | GHSV (s-l/kg catal•hr) | Productivity (g/mol/kg catal•hr) | | DME/MeOH Selectivity (mol%/mol%) |
|---|---|---|---|---|---|---|
| | | | | DME | MEOH | |
| 6 | Bal | 250 | 1870 | 2.46 | 0.96 | 72/28 |
| 7 | Bal | 250 | 3130 | 2.57 | 1.66 | 61/39 |
| 8 | Bal | 250 | 1120 | 1.92 | 0.57 | 77/23 |
| 9 | Bal | 263 | 1870 | 2.57 | 0.92 | 74/26 |
| 10 | Bal | 264 | 3730 | 3.09 | 1.45 | 68/32 |
| 11 | Bal | 265 | 1350 | 2.12 | 0.54 | 80/20 |
| 12 | CO-rich | 250 | 1870 | 1.41 | 0.42 | 77/23 |
| 13 | CO-rich | 250 | 1870 | 1.36 | 0.46 | 75/25 |
| 14 | CO-rich | 250 | 3130 | 1.18 | 0.59 | 66/34 |
| 15 | CO-rich | 250 | 1120 | 1.16 | 0.26 | 82/18 |

EXAMPLE III

A third series of experiments illustrates operation with $H_2O$ co-feed, and simultaneous shift, methanol and dimethyl ether reactions. A 15 wt% slurry comprised of 25 grams BASF K3-110 commercial low-temperature shift catalyst, 25 grams BASF S3-85, and 25 grams Catapal® alumina was slurried in 425 grams degassed Witco 70 oil. The pressure was 800 psig. The feed gas was 0.8% $H_2$, 57.7% CO, 15.5% $CO_2$, and balance $N_2$. Steam was co-fed with the gas to shift the CO and produce $H_2$. Results are summarized in Table 3.

Table 3

| Run | Feed Ratio $H_2O/CO$ | Temp (°C) | GHSV (s-1/kg catal•hr) | Productivity (gmol/kg catal•hr) | | DME/MeOH Selectivity (mol%/mol%) |
|---|---|---|---|---|---|---|
| | | | | DME | MeOH | |
| 16 | 0.50 | 249 | 2000 | 0.40 | 2.19 | 16/84 |
| 17 | 0.33 | 247 | 1860 | 2.64 | 2.11 | 56/44 |

EXAMPLE IV

The next series of experiments illustrates the use of a single catalyst species in the process. A copper on alumina catalyst was prepared by dissolving 64.03 grams $Cu(NO_3)_2$•$2.5H_2O$ in 100 ml deionized water. The solution was used to impregnate 78.14 grams $Al_2O_3$ in several portions, with $N_2$ purging between impregnations. The catalyst was dried overnight at 110°C, and reduced with 2% $H_2$ in $N_2$. Following reduction, 25 grams catalyst (equivilent to 40.6 gram as oxide) were slurried in 100 grams of degassed Witco 70 oil. The system was run at 800 psig, and results are shown in Table 4.

Table 4

| Run | Feed | Temp (°C) | GHSV (s-l/kg catal•hr) | Productivity (gmol/kg catal•hr) | | DME/MeOH Selectivity (mol%/mol%) |
|---|---|---|---|---|---|---|
| | | | | DME | MEOH | |
| 18 | Bal | 249 | 3000 | 0.58 | 0.45 | 56/44 |
| 19 | Bal | 265 | 3000 | 0.82 | 0.41 | 67/33 |
| 20 | Bal | 296 | 3000 | 1.07 | 0.28 | 79/21 |

EXAMPLE V

The last series of data illustrates the use of a catalyst mixture with a different methanol catalyst component. In two separate trials, 15 g BASF S3-86 catalyst and 15 g Davison silica alumina were slurried in 100 g of Sontex 100 mineral oil. The autoclave was pressurized to 750 psig with CO-rich gas. Results are summarized in Table 5.

Table 5

| Run | Temp ($^\circ$C) | GHSV (s-l/kg catal•hr) | Productivity (gmol/kg catal•hr) | | DME/MeOH Selectivity (mol%/mol%) |
|---|---|---|---|---|---|
| | | | DME | MEOH | |
| 21 | 250 | 1500 | 2.80 | 2.33 | 55/45 |
| 22 | 250 | 733 | 1.50 | 0.62 | 71/29 |
| 23 | 250 | 1500 | 2.73 | 1.32 | 67/33 |
| 24 | 250 | 1425 | 2.28 | 0.34 | 87/13 |
| 25 | 250 | 2163 | 1.61 | 1.81 | 47/53 |
| 26 | 260 | 1925 | 1.71 | 1.16 | 60/40 |
| 27 | 260 | 1500 | 2.42 | 0.89 | 73/23 |
| 28 | 260 | 733 | 0.56 | 0.21 | 73/23 |
| 29 | 260 | 1620 | 0.65 | 0.48 | 58/42 |
| 30 | 250 | 860 | 0.92 | 0.50 | 65/35 |

In the second of these trials with BASF S3-86, the effective rate of methanol production was deliberately decreased by injecting 4 g of chloride, as NaCl to the slurry. This decreased the effective rate of methanol production below the threshold value, and consequently decreased the dimethyl ether rate to nil.

Table 6

| Run | Temp ($^\circ$C) | GHSV (s-l/kg catal•hr) | Productivity (gmol/kg catal•hr) | | DME/MeOH Selectivity (mol%/mol%) |
|---|---|---|---|---|---|
| | | | DME | MEOH | |
| 31 | 250 | 2075 | 3.44 | 1.12 | 75/25 |
| 32 | 250 | 733 | 1.50 | 0.62 | 77/29 |
| 33 | 250 | 1500 | 2.73 | 1.32 | 67/33 |
| 34 | 250 | 2075 | 0.00 | 0.04 | 0/100 |
| 35 | 270 | 1300 | 0.00 | 0.00 | -- |

The foregoing examples demonstrate that a useful single step process for synthesis of dimethyl ether from synthesis gas requires two essential features. First, the effective rate of methanol synthesis must exceed a minimum threshold value. Second, it is essential to have a catalyst or catalyst mixture which incorporates both a methanol-forming component and a dehydration component.

Because methanol is the key intermediate reactant in this process, its rate of production has an important effect on the overall process performance. This point can be illustrated by defining the effective rate of methanol synthesis, $r^*_{M\,eOH}$ as:

$$r^*_{M\,eOH} = r_{MeOH} + 2\,(r_{DME})$$

where $r_{MeOH}$ and $r_{DME}$ are the rates of methanol and dimethyl ether formation, respectively, measured with respect to the total quantity of catalyst in the process.

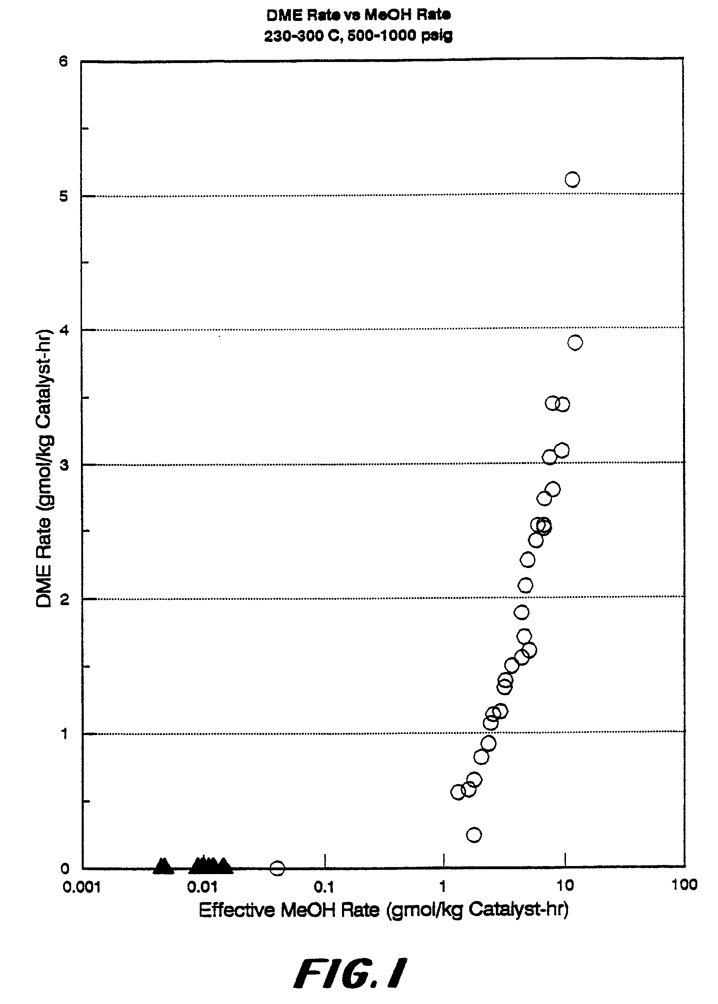

The present invention clearly demonstrates that there is a certain minimum value of $r^*_{M\,eOH}$ that is necessary in order to produce significant quantities of dimethyl ether. Below this threshold value, at best only trace quantities of dimethyl ether will be formed. Figure 1, which plots $r_{DME}$ versus $r^*_{M\,eOH}$ for the data of the present invention and Sherwin et al., shows that the minimum effective methanol rate ($r^*MeOH$) is about 1.0 gmol/(kg catalyst • hr). Thus, Figure 1 clearly explains why Sherwin et al., whose results are shown as closed triangles, where unsuccessful in their attempt to produce dimethyl ether. These experiments were all operated below the threshold limit for the production of intermediate methanol, and hence yielded only trace quantities of dimethyl ether. The results for the present invention, shown as open circles, confirm that the quantity of dimethyl ether produced is essentially nil at these low effective methanol rates. However, the

7

dimethyl ether rate increases rapidly once the minimum threshold rate is achieved. The data confirm this conclusion for the process using several different catalyst systems, feeding several synthesis gases with a wide range of compositions, and operating over a wide range of temperatures and pressures. Hence a useful commercial process to produce dimethyl ether from synthesis gas must operate at or above the threshold rate.

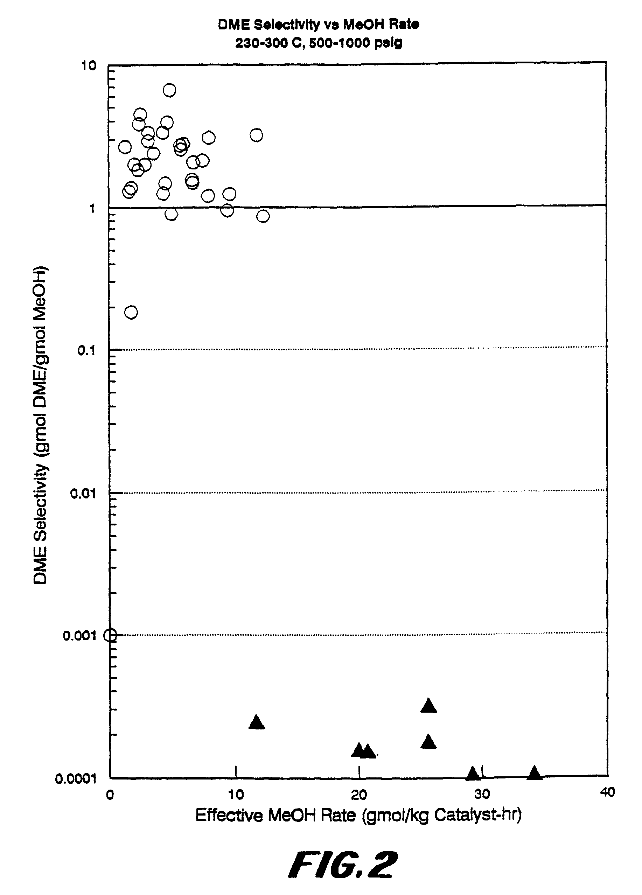

Achieving the threshold rate is the first essential aspect of our process. The second essential aspect is that the catalyst or catalyst mixture contain both a methanol forming component and a dehydration component. It is well known that all processes for production of methanol from synthesis gas yield trace quantities of dimethyl ether. However, these trace quantities are too small to constitute a commercially viable process for dimethyl ether. Dimethyl ether is produced in significant quantity only when both catalyst components are present. Figure 2 confirms this observation by comparing results for the liquid phase dimethyl ether process, shown as open circles, to the liquid phase methanol process, shown as closed triangle, at similar operating conditions. Dimethyl ether selectivity, defined as the moles of dimethyl ether produced divided by the moles of methanol produced, is shown as a function of the effective methanol rate. As discussed above, the amount of dimethyl ether produced is very low below the threshold rate. Above the threshold rate, dimethyl ether production is significant in our process, since the selectivity is greater than unity. However, dimethyl ether production is insignificant in the liquid phase methanol process over the entire range of operation because the dehydration catalyst component is not present.

In order to obtain the minimum effective methanol rate the traditional operating parameters, i.e., temperature, pressure, catalyst type, and oil type can be manipulated, however, there is an additional parameter which appears to have a significant effect on the amount of dimethyl ether produced by the process of the present invention. This variable is the particle size of the catalyst or catalyst components in the liquid medium. The earlier work by Sherwin et al. recites some catalysts of unspecified size and others with sizes from 0.85 to 1.20 mm; the work of the present invention has exclusively used catalysts with particle sizes below 200 microns, and preferably below 10 microns. The process of the present invention produced significant quantities of dimethyl ether; the process as taught by Sherwin et al. made only trace quantities of dimethyl ether.

The present invention with its efficient production of dimethyl ether with a methanol co-product can address a limitation of the liquid phase methanol process, which usually arises in its application for use in integrated gasification combined cycle electric power plants. In this application, the liquid phase methanol process generates methanol, a storable fuel, for peak-shaving. However, the methanol production rate is often limited by thermodynamic constraints. Hence, there is a limit to the amount of storable fuel which can be produced by the liquid phase methanol process. Co-production of dimethyl ether and methanol does not suffer from this thermodynamic constraint. Consequently, an improved process could produce a greater quantity of storable fuel.

The present invention solves the previously described problems through two features inherent in the process. First, the liquid medium acts as a heat sink, resulting in isothermal operation for the reactor. This factor is critical since the forward shift, methanol synthesis, and dimethyl ether synthesis reactions are all exothermic. With conventional gas-phase processes, the heat released during reaction increases temperature, which impedes reaction due to thermodynamic limitations, and causes catalyst deactivation. The high thermal capacity of the liquid phase permits high conversions while maintaining stable temperatures. This excellent temperature control may be responsible for increased catalyst life of this process relative to gas phase operations which require frequent catalyst regeneration.

The second feature of this process is that it uniquely exploits the synergism of the chemistry of all three reactions by combining them in a single step. By combining the three reactions in a simultaneous process, each of the individual reactions is driven thermodynamically by removing its inhibiting products as reactants for the subsequent reaction. For example, the first series of experiments showed CO conversions for the DME process which exceed the thermodynamic maximum for methanol synthesis alone. Such synergism cannot be achieved in multi-step process, where each reaction proceeds at most to its individual thermodynamic limitation in separate reactors.

Also, since all reactions in the process of the present invention proceed simultaneously, the process permits several options for varying the product distribution by manipulating the extent of each reaction. For example, the DME/MeOH selectivity can be controlled by varying the amount or activity of catalyst constituents in a process using a mixture of catalysts, or by altering the intrinsic selectivity of the catalyst in a process using a single catalyst. The product distribution can also be varied by changing reaction conditions such as space velocity, temperature, pressure or feed compositions.

As a summary, the distinguishing features of the present invention are the liquid phase operation and the concommitant occurrence of the shift, MeOH synthesis, and DME synthesis reactions. Both features are required for efficient operation and process flexibility.

Although several processes make use of combinations of reactions (1) through (3), the reactions are conducted in the gas phase. Since the shift and methanol synthesis reactions are thermodynamically limited by high temperatures and all three reactions are exothermic, removal of heat from the reactor is a critical and probably the limiting factor in their design. The significance of the thermal control provided by the liquid phase is best illustrated by comparing gas and liquid phase processes. For example, the adiabatic temperature rise for a gas-phase process providing the same conversions as the conditions of Run 1 is 350°C, versus a liquid phase process with an actual temperature rise less than 10°C due to the presence of the liquid phase. No current commercial catalyst could function economically at a gas phase adiabatic temperature of 600°C without heat removal equipment or product gas recycling. Both options are generally very expensive. For example, using product gas recycling to control the temperature rise would require a recycle ratio in the range of 10 to 20. Such high recycle ratios require high capital investments for compressors and reactors, as well as high operating costs. In comparison, a liquid phase unit would require little or no feed recycle, and a much smaller reactor. Hence liquid phase synthesis can provide economic operation at high conversions.

It should be noted that the prior art has erroneously concluded that liquid phase operation, such as the process of this invention, does not work; Sherwin et al. failed to produce DME in systems very similar to this process. Hence the success of this process is both novel and surprising. The only other liquid phase synthesis discussed in the prior art is non-selective and consumes an expensive solvent as a reagent, see Daroda et al.

The second distinguishing feature of the invention, the simultaneous shift, methanol synthesis, and DME synthesis reactions, enable the process to use feeds with wide ranges in composition. Previously disclosed processes can only operate within restricted ranges of $H_2/CO$ or $CO/CO_2$ ratios. This invention demonstrates operation with feeds richer in CO than any previous processes. For example, Runs 16 and 17 show high productivity with feed CO concentrations of 58% and a $H_2/CO$ ratio below 0.02. Such conditions are well beyond those claimed or taught for the prior art processes.

The present invention has been described with reference to several embodiments thereof. These embodiments should not be considered limitations on the scope of the present invention, the scope of which should be ascertained from the following claims.

## Claims

1. In a process for the synthesis of dimethyl ether from a synthesis gas comprising hydrogen, carbon monoxide and carbon dioxide, wherein the synthesis gas is reacted in the presence of a solid methanol synthesis catalyst to produce methanol and wherein the produced methanol is reacted in the presence of a solid dehydration catalyst to produce dimethyl ether, the improvement comprising contacting and reacting the synthesis gas in the presence of a solid catalyst system comprising a methanol synthesis component and a dehydration (ether forming) component, wherein the solid catalyst system is a single catalyst or a mixture of catalysts in a liquid medium in a three phase (liquid phase) reactor system, and operating the reactor system so as to maintain a minimum effective methanol rate of at least 1.0 gmol methanol/(kg catalyst ● hr).

2. The process of Claim 1 wherein the catalyst is in the form of shaped pellets and the three phase reactor is operated in an ebullated mode.

3. The process of Claim 1 wherein the catalyst is in powdered form having a particle size of less than 200 microns and the three phase reactor is operated in a slurry mode.

4. The process of Claim 1 which further comprises operating the process at a pressure in the range from about 200 psig to about 2000 psig.

5. The process of Claim 1 which further comprises operating the process at a pressure in the range from about 400 psig to about 1000 psig.

6. The process of Claim 1 which further comprises operating the process at a temperature in the range from about 200°C to about 350°C.

7. The process of Claim 1 which further comprises operating the process at a space velocity in excess of 50 standard liters of synthesis gas per kilogram of catalyst per hour.

8. The process of Claim 1 which further comprises operating the process at a space velocity in the range from about 1,000 to about 20,000 standard liters of synthesis gas per kilogram of catalyst per hour.

9. The process of Claim 1 wherein the concentration of carbon monoxide in the synthesis gas is in excess of 50 vol%.

10. The process of Claim 1 which further comprises adding water to the three phase reactor as a co-feed.

11. The process of Claim 10 wherein water is added as a liquid.

12. The process of Claim 10 wherein the concentration of hydrogen in the synthesis gas is less than 10 vol%.

13. The process of Claim 1 wherein the concentration of catalyst in the liquid medium is in the range from about 5 wt% to about 60 wt%.

14. The process of Claim 1 wherein the catalyst system is a single catalyst containing both a methanol synthesis component and a dehydration (ether forming) component.

15. The process of Claim 1 wherein the methanol synthesis catalyst component comprises copper.

16. The process of Claim 1 wherein the dehydration (ether forming) catalyst component is selected from the group consisting of alumina, silica-alumina, zeolites, solid acids, solid acid ion exchange resins and mixtures thereof.

17. The process of Claim 1 which further comprises removing carbon dioxide from the synthesis gas prior to contacting and reacting the synthesis gas with the solid catalyst.

18. In a process for the direct synthesis of dimethyl ether and methanol co-products from a synthesis gas comprising hydrogen, carbon monoxide and carbon dioxide, wherein the synthesis gas is contacted with and reacted in the presence of a solid catalyst, the improvement comprising contacting and reacting the synthesis gas in the presence of a solid catalyst system, wherein the solid catalyst system is a single catalyst or a mixture of catalysts, suspended in a liquid medium in a three phase (liquid phase) reactor system, wherein the three phase reactor system comprises at least one three phase reactor.

19. In a process for the generation of electricity by an integrated gasifier combined cycle power plant, wherein a synthesis gas is contacted with and reacted in the presence of a solid catalyst to produce a storable fuel for peak-shaving, the improvement for increasing the amount of storable fuel which comprises producing a dimethyl ether co-product in addition to methanol by contacting and reacting the synthesis gas in the presence of a solid catalyst system, wherein the solid catalyst system is a single catalyst or a mixture of catalysts, suspended in a liquid medium in a three phase (liquid phase) reactor system, wherein the three phase reactor system comprises at least one three phase reactor.

FIG. I

DME Selectivity vs MeOH Rate
230-300 C, 500-1000 psig

*FIG.2*

## DOCUMENTS CONSIDERED TO BE RELEVANT

EP 90113362.9

| Category | Citation of document with indication, where appropriate, of relevant passages | Relevant to claim | CLASSIFICATION OF THE APPLICATION (Int. Cl.5) |
|---|---|---|---|
| P,X | EP - A1 - 0 324 475 (AIR PRODUCTS AND CHEMICALS) * Claims 1-20 * | 1-... | C 07 C 43/04 C 07 C 41/01 |
| D,A | US - A - 4 590 176 (AREND HOEK et al.) * Abstract * | 1, 14. | |
| D,A | US - A - 4 521 540 (RONALD PIERANTOZZI) * Abstract * | 1, 14-16 | |
| A | DE - A1 - 3 220 547 (SNAMPROGETTI) * Claim 1 * | 1 16 | |
| A | GB - A - 2 097 382 (MOBIL OIL) * Abstract * | 1, 1. | |
| A | GB - A - 278 353 (DELCO-LIGHT) * Page 1, lines 37-65; page 2, lines 21-32 * | 1,.. 1.. | TECHNICAL FIELDS SEARCHED (Int. Cl.5) C 07 C 43/00 C 07 C 41/00 |
| P,A | EP - A2 - 0 343 454 (UNION RHEINISCHE BRAUN-KOHLEN) * Abstract * | 1 | C 07 C 27/00 C 07 C 1/00 |

The present search report has been drawn up for all claims

| Place of search | Date of completion of the search | Examiner |
|---|---|---|
| VIENNA | 18-10-1990 | IF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the
L : document cited for u...

& : member of the same pa... ...nily, corresponding document

EPO FORM 1503 03.82 (P0401)